# EUROPEAN PATENT APPLICATION

(11) **EP 4 332 582 A1**
(43) Date of publication of application: **06.03.2024**
(21) Application number: 22794205.9
(22) Date of filing: 19.01.2022
(51) Int. Cl.: G01N 35/04

(54) **REAGENT TRANSFER DEVICE AND IMMUNOLOGICAL DIAGNOSTIC APPARATUS**

(30) Priority: 28.04.2021 CN 202110466625
(71) Applicant: Shenzhen Yhlo Biotech Co., Ltd, 518116 Shenzhen (CN)
(72) Inventor: MO, Shaohao, Shenzhen, Guangdong 518116 (CN); HUANG, Xiutao, Shenzhen, Guangdong 518116 (CN); ZHANG, Fuxing, Shenzhen, Guangdong 518116 (CN); XIAO, Yujin, Shenzhen, Guangdong 518116 (CN)
(74) Representative: Mewburn Ellis LLP
(86) International application number: PCT/CN2022/072669
(87) International publication number: WO 2022/227723

(57) **Abstract**

A reagent transfer device (10) and an immunological diagnostic apparatus. The reagent transfer device (10) comprises a transfer supporting mechanism (100) and reagent transfer mechanisms (200), wherein a plurality of reagent transfer mechanisms (200) are provided, and the plurality of reagent transfer mechanisms (200) are respectively mounted to the transfer supporting mechanism (100); and each reagent transfer mechanism (200) comprises a transfer base (210), a reagent needle (220) and a multidirectional movement component (230), the transfer base (210) being mounted to the transfer support mechanism (100), the multidirectional movement component (230) being arranged on the transfer base (210), and the multidirectional movement component (230) being connected to the reagent needle (220) so as to drive the reagent needle (220) to move. The immunological diagnostic apparatus comprises the reagent transfer device (10). The reagent transfer device (10) can solve the problems of the low reagent transfer efficiency and low detection efficiency of a chemiluminescence immunoassay system in the prior art.

## Description

### TECHNICAL FIELD

The present application relates to the fields of biological detection and diagnosis, and in particular, to a reagent transfer device and an immunological diagnostic apparatus.

### BACKGROUND

With the development of modern science, immunodiagnostic technology has gradually become a focus means of modern clinical testing and scientific research. Immunodiagnostic technology is an important diagnostic means for tumor, diabetes, abnormal gonadal secretion and other diseases. Compared with conventional detection methods having the drawbacks of complex operation, long reaction time, environmental pollution, etc., the immunodiagnostic technology such as chemiluminescent immunoassay technology has the advantages of short reaction time, simple operation, high diagnostic efficiency and the like, and it has been widely applied in modern medicine and scientific research fields.

At present, a chemiluminescent immunoassay system mainly includes: a cuvette storage and transfer system, a sample loading system, a reagent loading system, a sample dispensing system, an incubation system, a centrifugal wash system, and a luminescent reading system. In the sample dispensing system, when transferring a reagent, the reagent placed in a refrigerated reagent box of a reagent plate is dispensed to a cuvette of the incubation system. Both the reagent plate and the incubation system need to wait for the reagent transfer system to complete the previous reagent transfer before transferring the next reagent. There is a gap waiting period in the timing sequence, which causes time waste and low detection efficiency.

### SUMMARY

Accordingly, it is necessary to provide a reagent transfer device and an immunological diagnostic apparatus, which can solve the problems of low reagent transfer efficiency and low detection efficiency of the chemiluminescent immunoassay system in the prior art.

A reagent transfer device includes a transfer support mechanism and a plurality of reagent transfer mechanisms. The plurality of reagent transfer mechanisms are respectively mounted on the transfer support mechanism. Each of the plurality of reagent transfer mechanisms includes a transfer base, a reagent needle, and a multi-directional moving part. The transfer base is mounted on the transfer support mechanism. The multi-directional moving part is disposed on the transfer base. The multi-directional moving part is connected to the reagent needle to drive the reagent needle to move.

In an embodiment, the multi-directional moving part includes a first guide element, a second guide element, a first driver, and a second driver. The first guide element is mounted on the transfer base and extends in a first direction. The second guide element is slidably connected to the first guide element and extends in a second direction. The first driver is connected to the second guide element for driving the second guide element to move along the first guide element. The reagent needle is slidably connected to the second guide element. The second driver is mounted on the second guide element and connected to the reagent needle. The second driver is configured to drive the reagent needle to move along the second guide element.

In an embodiment, the first direction is a horizontal direction, the second direction is a vertical direction, and the first direction is perpendicular to the second direction.

In an embodiment, the multi-directional moving part further includes a first synchronous belt, a first driving wheel, and a first driven wheel. The first driving wheel is connected to a drive shaft of the first driver. The first driven wheel is rotatably connected to the transfer base. The first driving wheel is connected to the first driven wheel through the first synchronous belt. The second guide element is connected to the first synchronous belt and moves with the first synchronous belt.

In an embodiment, the multi-directional moving part further includes a second synchronous belt, a second driving wheel, and a second driven wheel. The second driving wheel is connected to a drive shaft of the second driver. The second driven wheel is rotatably connected to the first guide element. The second driving wheel is connected to the second driven wheel through the second synchronous belt. The reagent needle is connected to the second synchronous belt through a needle base and moves with the second synchronous belt.

In an embodiment, a plurality of reagent needles are provided. The plurality of reagent needles are arranged at intervals.

In an embodiment, the reagent transfer mechanism further includes a washing well. The washing well is connected to the transfer base and configured to wash the plurality of reagent needles.

In an embodiment, the number of washing wells is the same as the number of reagent needles. A plurality of washing wells are distributed at intervals. A distribution order of the plurality of washing wells is the same as that of the plurality of reagent needles.

In an embodiment, a position of the reagent transfer mechanism on the transfer support mechanism is adjustable.

The immunological diagnostic apparatus includes the above reagent transfer device.

The above reagent transfer device can solve the problems of low reagent transfer efficiency and low detection efficiency of the chemiluminescent immunoassay system in the prior art. The reagent transfer device can realize the synchronous or cross transfers of multiple reagents. When one reagent transfer mechanism performs transfer of reagent, another reagent transfer mechanism can prepare for another transfer of reagent, and perform this another transfer of reagent after the transfer of reagent of the previous reagent transfer mechanism is completed. Alternatively, the plurality of reagent transfer mechanisms perform synchronous or cross transfers of multiple reagents, and each reagent transfer mechanism can operate in linkage or independently.

The above reagent transfer device is provided with the first guide element and the second guide element, which limits the moving direction of the reagent needle, realizes the movements of the reagent needle in the first direction and the second direction, and improves the moving accuracy of the reagent needle.

By setting the first direction as the horizontal direction, the second direction as the vertical direction, and the first direction perpendicular to the second direction, the above reagent transfer device can realize the movements of the reagent needle in the horizontal direction and the vertical direction. The reagent needle can be transferred in the horizontal direction between the upstream and downstream positions, such as between the incubation plate device and the reagent needle plate device, and can rapidly draw and inject reagent, and enter the washing well to be washed in the vertical direction.

The above reagent transfer device achieves stable movement of the second guide element by providing the first synchronous belt, the first driving wheel and the first driven wheel, thereby improving the movement stability of the second guide element.

The above reagent transfer device achieves stable movement of the reagent needle by providing the second synchronous belt, the second driving wheel and the second driven wheel, thereby improving the movement stability of the reagent needle.

By providing the plurality of reagent needles and arranging the plurality of reagent needles at intervals, the above reagent transfer device can realize the synchronous operation of two reagent needles and satisfy the transfer of multiple reagents.

The reagent transfer device described above can be configured to wash the reagent needle by arranging the washing well. After transferring the reagent, the reagent needle can enter the washing well to be washed, which can facilitate the transfer of the next reagent or the transfer of another type of reagent.

In summary, the above reagent transfer device has the following advantages.
(1) The plurality of reagent transfer mechanisms can all realize linear movement of the reagent needle, and the reagent transfer device is mounted between the incubation plate device and the reagent needle plate device, so that the immunological diagnostic apparatus has more compact layout, the structure is simple, the moving efficiency is high, and the space utilization rate is high.
(2) The plurality of reagent transfer mechanisms can operate simultaneously, which can reduce the waiting time at upstream and downstream positions, such as the waiting time of the incubation plate device and the reagent plate device, of the reagent transfer device, and improve the detection efficiency.
(3) Each of the reagent transfer mechanisms in the reagent transfer device can move the respective reagent needles to respective dispensing stations at the upstream and downstream positions, such as respective dispensing stations of the incubation plate device and the reagent plate device, so that the reagent needle draws and injects reagent quickly, the reagent transfer efficiency is improved, the detection time is saved, and the detection efficiency is improved.
(4) The reagent transfer mechanism and the transfer support mechanism are removable from each other so that the separation the reagent transfer mechanism and the transfer support mechanism can be achieved, so as to increase maintainability.
(5) The reagent transfer mechanism is provided with the washing well, which is configured to wash the reagent needle, so that the reagent needle can automatically draw and inject reagent, and be automatically washed.

### BRIEF DESCRIPTION OF THE DRAWINGS

FIG. 1 is a schematic diagram of a reagent transfer device according to an embodiment of the present application.
FIG. 2 is a schematic diagram of a reagent transfer device according to an embodiment of the present application.
FIG. 3 is a schematic diagram of a reagent transfer mechanism, which is partially disassembled, according to an embodiment of the present application.
FIG. 4 is a schematic diagram illustrating a cooperation between a reagent transfer device and an incubation plate device, and between the reagent transfer device and a reagent needle plate device according to an embodiment of the present application.

Illustration For Reference Numerals:
10, reagent transfer device; 100, transfer support mechanism; 200, reagent transfer mechanism; 210, transfer base; 220, reagent needle; 230, multi-directional moving part; 231, first guide element; 232, second guide element; 233, first driver; 234, second driver; 235, first synchronous belt; 236, second synchronous belt; 237, second driving wheel; 240, washing well; 20, incubation plate device; and 30, reagent needle plate device.

### DETAILED DESCRIPTION OF THE EMBODIMENTS

In order to make the objects, features, and advantages of the present application more apparent and understandable, the specific implementations of the present application will be illustrated in detail below in conjunction with the accompanying drawings. In the following description, numerous specific details are set forth in order to facilitate a thorough understanding of the present application. However, the present application can be implemented in many other ways different from those described herein, and those skilled in the art can make similar modifications without departing from the connotation of the application, so that the present application is not limited to the specific embodiments disclosed below.

In the description of the present application, it is to be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. indicate the orientations or positional relationships on the basis of the drawings. These terms are only intended for facilitating illustrating the present application and simplifying the illustration, rather than indicating or implying that the devices or elements referred thereto have to present particular orientations, and be constructed and operated in particular orientations, and therefore cannot be construed as limiting the present application.

In addition, the terms "first" and "second" are only intended for illustrative purposes, rather than being construed as indicating or implying relative importance or implicitly designating the number of the technical features as indicated. Thus, the features defined by "first" and "second" may explicitly or implicitly include at least one of the said features. In the illustrations of the present application, the term "a plurality of" means at least two, for example two or three, unless otherwise explicitly and specifically defined.

In the present application, unless otherwise expressly specified and defined, the terms "mounted", "connected to", "coupled" and "fixed" should be understood in a broad sense, for example, fixedly connected or detachably connected, or integrated; mechanically connected or electrically connected; directly connected or indirectly connected through an intermediate medium, or in an interior communication or mutual interaction relationship between two elements, unless otherwise specifically defined. For those of ordinary skill in the art, the specific meanings of the above-described terms in the present application may be understood according to specific circumstances.

In the present application, unless otherwise expressly stated and defined, the first feature, when being referred to as being located "above" or "below" the second feature, may be in direct contact with the second feature, or in indirect contact with the second feature via an intermediate feature. Moreover, the first feature, when being referred to as being disposed "on", "above" and "over" the second feature, may be disposed right above or obliquely above the second feature, or simply disposed at a level higher than the second feature. The first feature, when being referred to as being disposed "under", "below" and "beneath" the second feature, may be disposed directly below or obliquely below the second feature, or simply disposed at a level lower than the second feature.

It should be noted that, when an element is referred to as being "fixed to" or "disposed on" another element, it may be directly on the other element or there may also be present an intermediate element. When an element is referred to as being "connected" to another element, it may be directly connected to the other element or there might be present an intermediate element at the same time. The terms "vertical", "horizontal", "up", "down", "left", "right" and similar expressions used herein are only for the purpose of illustration, rather than presenting the only ways for implementation.

Unless otherwise defined, all technical and scientific terms used herein have the same meanings as those commonly understood by those skilled in the art to which the present application belongs. The terms used in the description of the present application herein are only for the purpose of describing specific embodiments and are not intended to limit the present application. The term "and/or" used herein includes any and all combinations of one or more associated listed items.

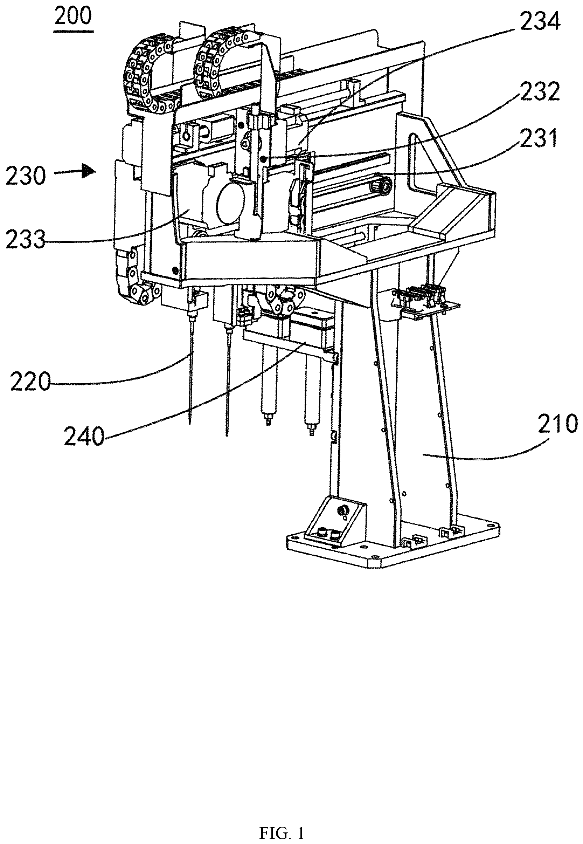

Referring to FIG. 1, an embodiment of the present application provides a reagent transfer device 10.

The reagent transfer device 10 includes a transfer support mechanism 100 and a reagent transfer mechanism 200. The support mechanism may be of a long strip structure. When in use, the support mechanism is provided between an incubation plate device 20 and a reagent needle plate device 30. The support mechanism extends between the incubation plate device 20 and the reagent needle plate device 30 to enable the reagent transfer mechanism 200 to transfer the reagent between the incubation plate device 20 and the reagent needle plate device 30.

A plurality of reagent transfer mechanisms 200 are provided. The plurality of reagent transfer mechanisms 200 are respectively mounted on the transfer support mechanism 100.

The reagent transfer mechanism 200 includes a transfer base 210, a reagent needle 220, and a multi-directional moving part 230. The transfer base 210 is mounted on the transfer support mechanism 100. The multi-directional moving part 230 is disposed on the transfer base 210. The multi-directional moving part 230 is connected to the reagent needle 220 for driving the reagent needle 220 to move.

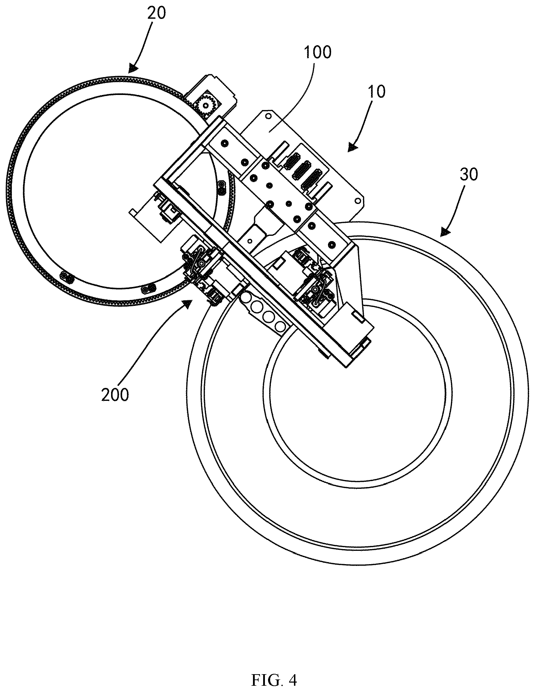

In an embodiment, two, three, four, etc., reagent transfer mechanisms 200 are provided. The number of reagent transfer mechanisms 200 is not limited to the above. It will be appreciated that in other embodiments, other quantities of reagent transfer mechanisms 200 may also be provided. Preferably, two reagent transfer mechanisms 200 are provided. As shown in FIG. 4, the two reagent transfer mechanisms 200 are disposed on the support mechanism. The two reagent transfer mechanisms 200 are capable of transferring the reagent between the incubation plate device 20 and the reagent needle plate device 30.

In an embodiment, the multi-directional moving part 230 includes a first guide element 231, a second guide element 232, a first driver 233, and a second driver 234.

The first guide element 231 is mounted on the transfer base 210 and extends in a first direction. The second guide element 232 is slidably connected to the first guide element 231 and extends in a second direction. The first driver 233 is connected to the second guide element 232 to drive the second guide element 232 to move along the first guide element 231. The reagent needle 220 is slidably connected to the second guide element 232. The second driver 234 is mounted on the second guide element 232 and connected to the reagent needle 220. The second driver 234 is configured to drive the reagent needle 220 to move along the second guide element 232. The above reagent transfer device 10 is provided with the first guide element 231 and the second guide element 232, which limits the moving direction of the reagent needle 220, realizes the movements of the reagent needle 220 in the first direction and the second direction, and improves the moving accuracy of the reagent needle 220.

In an embodiment, the first guide element 231 is a first guide rail. The second guide element 232 is a second guide rail. The first driver 233 is a first motor, and the second driver 234 is a second motor.

In an embodiment, the first direction is a horizontal direction, the second direction is a vertical direction, and the first direction is perpendicular to the second direction. By setting the first direction as the horizontal direction, the second direction as the vertical direction, and the first direction perpendicular to the second direction, the above reagent transfer device 10 can realize the movements of the reagent needle 220 in the horizontal direction and the vertical direction. The reagent needle 220 can be transferred in the horizontal direction between the upstream and downstream positions, such as between the incubation plate device 20 and the reagent needle plate device 30, and can rapidly draw and inject reagent, and enter a washing well 240 to be washed in the vertical direction.

In an embodiment, the multi-directional moving part 230 further includes a first synchronous belt 235, a first driving wheel, and a first driven wheel. The first driving wheel is connected to a drive shaft of the first driver 233. The first driven wheel is rotatably connected to the transfer base 210. The first driving wheel is connected to the first driven wheel through the first synchronous belt 235. The second guide element 232 is connected to the first synchronous belt 23 5 and moves with the first synchronous belt 235. The above reagent transfer device 10 achieves stable movement of the second guide element 232 by providing the first synchronous belt 235, the first driving wheel and the first driven wheel, thereby improving the movement stability of the second guide element 232.

In an embodiment, the multi-directional moving part 230 further includes a second synchronous belt 236, a second driving wheel 237, and a second driven wheel. The second driving wheel 237 is connected to a drive shaft of the second driver 234. The second driven wheel is rotatably connected to the first guide element 231. The second driving wheel 237 is connected to the second driven wheel through the second synchronous belt 236. The reagent needle 220 is connected to the second synchronous belt 236 through a needle base and moves with the second synchronous belt 236. The above reagent transfer device 10 achieves stable movement of the reagent needle 220 by providing the second synchronous belt 236, the second driving wheel 237 and the second driven wheel, thereby improving the movement stability of the reagent needle 220.

Preferably, the second synchronous belt 236 is a toothed rack. The second driving wheel 237 and the second driven wheel are both gears. The second driving wheel 237 and the second driven wheel respectively mesh with the second synchronous belt 236.

In an embodiment, a plurality of reagent needles 220 are provided. The plurality of reagent needles 220 are arranged at intervals. By providing the plurality of reagent needles 220 and arranging the plurality of reagent needles 220 at intervals, the above reagent transfer device 10 can realize the synchronous operation of two reagent needles 220 and satisfy the transfer of multiple reagents.

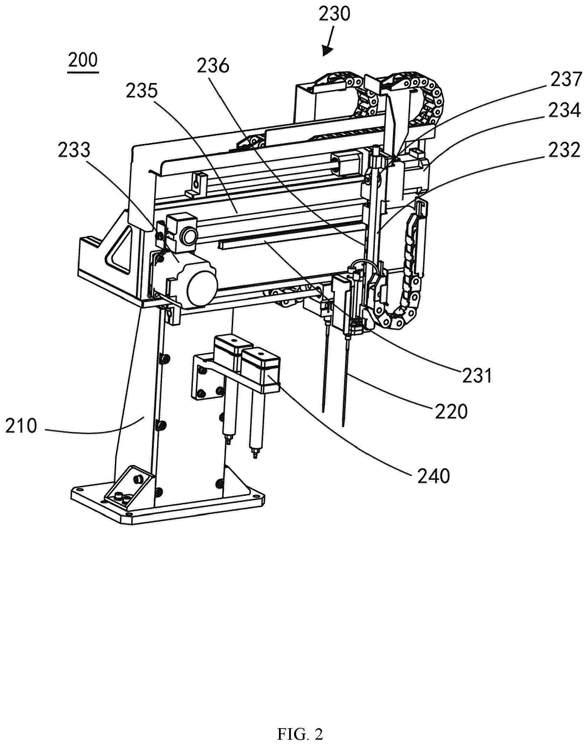

For example, in a specific embodiment, two, three, four, etc., reagent needles 220 are provided. The number of reagent needle 220 is not limited to the above. It will be appreciated that in other embodiments, other quantities of reagent needles 220 may also be provided. Preferably, two reagent needles 220 are provided. As shown in FIGS. 1 and 2, the reagent needles 220 are arranged in parallel, and there is an interval between the reagent needles 220.

In an embodiment, the reagent transfer mechanism 200 further includes the washing well 240. The washing well 240 is connected to the transfer base 210 and configured to wash the reagent needle 220. The reagent transfer device 10 described above can be configured to wash the reagent needle 220 by arranging the washing well 240. After transferring the reagent, the reagent needle 220 can enter the washing well 240 to be washed, which can facilitate the transfer of the next reagent or the transfer of another type of reagent.

In an embodiment, the number of washing wells 240 is the same as the number of reagent needles 220. A plurality of washing wells 240 are distributed at intervals. The distribution order of the washing wells 240 is the same as that of the reagent needles 220. When two reagent needles 220 are provided, two washing wells 240 are provided. The two washing wells 240 are arranged at an interval. The interval between the two washing wells 240 is equal to the interval between the two reagent needles 220. The washing wells 240 are arranged at positions according to the following requirements: the washing wells 240 are mounted on the transfer base 210, and the heights of the washing wells 240 on the transfer base 210 are required to ensure that the reagent needles 220 can be inserted into the washing wells 240 and be in contact with the bottom of the washing well 240; and the height of the washing wells 240 are required to be less than the maximum heights that the reagent needles 220 can be raised to ensure that the reagent needles 220 can enter the washing wells 240 respectively.

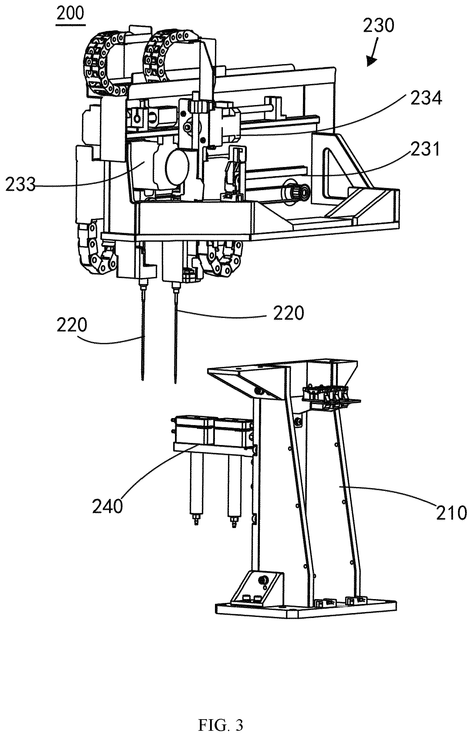

In an embodiment, the position of the reagent transfer mechanism 200 on the transfer support mechanism 100 is adjustable. The reagent transfer mechanism 200 and the transfer support mechanism 100 may be detachably connected to each other, for example, the reagent transfer mechanism 200 and the transfer support mechanism 100 are connected by fastening screws, or are in an engagement connection, in a sleeved connection, or the like. Preferably, as shown in FIG. 3, a detachable connection is achieved between the transfer base 210 of the reagent transfer mechanism 200 and the transfer support mechanism 100 by the fastening screws.

An immunological diagnostic apparatus including the reagent transfer device 10 is provided.

The above reagent transfer device 10 solves the problems of low reagent transfer efficiency and low detection efficiency of the chemiluminescent immunoassay system in the prior art. The reagent transfer device 10 can realize the synchronous or cross transfers of multiple reagents. When one reagent transfer mechanism 200 performs transfer of reagent, another reagent transfer mechanism 200 can prepare for another transfer of reagent, and perform this another transfer of reagent after the transfer of reagent of the previous reagent transfer mechanism 200 is completed. Alternatively, the plurality of reagent transfer mechanisms 200 perform synchronous or cross transfers of multiple reagents, and each reagent transfer mechanism 200 can operate in linkage or independently.

The reagent transfer device 10 has the following advantages.
(1) The plurality of reagent transfer mechanisms 200 can all realize linear movements of the reagent needles 220, and the reagent transfer device 10 is mounted between the incubation plate device 20 and the reagent needle plate device 30, so that the immunological diagnostic apparatus has a more compact layout, the structure is simple, the moving efficiency is high, and the space utilization rate is high.
(2) The plurality of reagent transfer mechanisms 200 can operate simultaneously, which can reduce the waiting time at the upstream and downstream positions, such as the waiting time of the incubation plate device 20 and the reagent plate device, of the reagent transfer device 10, and improve the detection efficiency.
(3) Each of the reagent transfer mechanisms 200 in the reagent transfer device 10 can move the respective reagent needles 220 to respective dispensing stations at the upstream and downstream positions, such as respective dispensing stations of the incubation plate device 20 and the reagent plate device, so that the reagent needle 220 draws and injects reagent quickly, the reagent transfer efficiency is improved, the detection time is saved, and the detection efficiency is improved.
(4) The reagent transfer mechanism 200 and the transfer support mechanism 100 are removable from each other so that the separation of the reagent transfer mechanism 200 and the transfer support mechanism 100 can be achieved, so as to increase maintainability.
(5) The reagent transfer mechanism 200 is provided with the washing well 240, which is configured to wash the reagent needle 220, so that the reagent needle 220 can automatically draw and inject reagent, and be automatically washed.

The technical features of the embodiments above may be combined arbitrarily. To make the description concise, not all possible combinations of the technical features in the above embodiments are described. However, as long as there are no contradictions in the combinations of these technical features, all of the combinations should be considered to be within the scope of the specification.

The embodiments above only represent several implementation modes of the present application, and the description thereof is relatively specific and detailed, but it should not be construed as limiting the scope of the patent. It should be noted that for those skilled in the art, various modifications and improvements may be made without departing from the concept of the present application, and all these modifications and improvements fall within the protection scope of the present application. Therefore, the scope of protection of the patent application shall be subject to the appended claims.

## Claims

1. A reagent transfer device, **characterized by**, comprising:
a transfer support mechanism; and
a plurality of reagent transfer mechanisms respectively mounted on the transfer support mechanism,
wherein each of the plurality of reagent transfer mechanisms comprises:
a transfer base mounted on the transfer support mechanism,
a reagent needle, and
a multi-directional moving part disposed on the transfer base, and connected to the reagent needle to drive the reagent needle to move.

2. The reagent transfer device according to claim 1, wherein the multi-directional moving part comprises a first guide element, a second guide element, a first driver, and a second driver,
wherein the first guide element is mounted on the transfer base and extends in a first direction; the second guide element is slidably connected to the first guide element and extends in a second direction; the first driver is connected to the second guide element to drive the second guide element to move along the first guide element; the reagent needle is slidably connected to the second guide element; the second driver is mounted on the second guide element and connected to the reagent needle; and the second driver is configured to drive the reagent needle to move along the second guide element.

3. The reagent transfer device according to claim 2, wherein the first direction is a horizontal direction, the second direction is a vertical direction, and the first direction is perpendicular to the second direction.

4. The reagent transfer device according to claim 2, wherein the multi-directional moving part further comprises a first synchronous belt, a first driving wheel, and a first driven wheel,
wherein the first driving wheel is connected to a drive shaft of the first driver; the first driven wheel is rotatably connected to the transfer base; the first driving wheel is connected to the first driven wheel through the first synchronous belt; and the second guide element is connected to the first synchronous belt and capable of moving with the first synchronous belt.

5. The reagent transfer device according to any one of claims 2 to 4, wherein the multi-directional moving part further comprises a second synchronous belt, a second driving wheel, and a second driven wheel,
wherein the second driving wheel is connected to a drive shaft of the second driver; the second driven wheel is rotatably connected to the first guide element; the second driving wheel is connected to the second driven wheel through the second synchronous belt; and the reagent needle is connected to the second synchronous belt through a needle base and moves with the second synchronous belt.

6. The reagent transfer device according to any one of claims 1 to 4, wherein a plurality of reagent needles are provided; and the plurality of reagent needles are arranged at intervals.

7. The reagent transfer device according to claim 6, wherein the reagent transfer mechanism further comprises a washing well connected to the transfer base and configured to wash the plurality of reagent needles.

8. The reagent transfer device according to claim 7, wherein the number of washing wells is the same as the number of reagent needles; a plurality of washing wells are distributed at intervals, and a distribution order of the plurality of washing wells is the same as that of the plurality of reagent needles.

9. The reagent transfer device according to any one of claims 1 to 4, wherein a position of the reagent transfer mechanism on the transfer support mechanism is adjustable.

10. A immunological diagnostic apparatus, **characterized by**, comprising the reagent transfer device according to any one of claims 1 to 9.
